# EUROPÄISCHE PATENTSCHRIFT

(11) **EP 2 462 217 B1**
(45) Veröffentlichungstag und Bekanntmachung des Hinweises auf die Patenterteilung: **25.02.2015**
(21) Anmeldenummer: 10737944.8
(22) Anmeldetag: 06.08.2010
(51) Int. Cl.: C12M 1/00

(54) **BIOREAKTOR MIT SILICONBESCHICHTUNG**
BIOREACTOR COMPRISING A SILICON COATING
BIORÉACTEUR À REVÊTEMENT DE SILICONE

(30) Priorität: 07.08.2009 DE 102009028338
(43) Veröffentlichungstag der Anmeldung: 13.06.2012
(73) Patentinhaber: Wacker Chemie AG, 81737 München (DE)
(72) Erfinder: MÜLLER-REES, Christoph, 82049 Pullach (DE); PFALLER, Rupert, 80639 München (DE)
(74) Vertreter: Schuderer, Michael
(86) Internationale Anmeldenummer: PCT/EP2010/061491
(87) Internationale Veröffentlichungsnummer: WO 2011/015655

(56) Entgegenhaltungen:
- WO-A1-2008/145719
- DE-A1- 19 850 607
- US-A1- 2005 227 092
- US-A1- 2007 048 859
- US-A1- 2009 011 492

## Beschreibung

Die Erfindung betrifft einen Bioreaktor, welcher mit einer anwuchshemmenden Beschichtung ausgerüstet ist, Verfahren zur Ausrüstung von Bioreaktoren mit einer anwuchshemmenden Beschichtung, sowie die Verwendung von Siliconen zur Ausrüstung von Bioreaktoren mit einer anwuchshemmenden Beschichtung.

Die wirtschaftliche Kultivierung phototropher Mikroorganismen (Mikroalgen, Cyanobakterien, Purpurbakterien) im großtechnischen Maßstab ist aufgrund der Problematiken der Lichtversorgung, der monoseptischen Kulturführung sowie der Maßstabsvergrößerung bisher nicht gelöst. Ein universelles Standardsystem zur Kultivierung phototropher Mikroorganismen im Großmaßstab ist bis dato nicht verfügbar. Von mehreren 10.000 Vertretern phototropher Mikroorganismen werden heute lediglich einige wenige Dutzend in größeren Mengen hergestellt, wobei diese meist in offenen, nicht kontaminationsfreien Systemen produziert werden. Die Kultivierungsbedingungen phototropher Mikroorganismen, die im Pilotmaßstab in geschlossenen Reaktoren hergestellt werden, sind bis dato über einen längeren Zeitraum nicht konstant zu halten, da die in einer Kultivierungsphase sich bildenden phototrophen Mikroorganismen an den Reaktorwänden ablagern, was zu Schwankungen der eingetragenen Lichtmenge in das Kulturmedium sowie zu einer unsteten Durchmischung des Kulturmediums führt. Algenanlagerungen werden häufig durch Stressbedingungen (z. B. durch Scherung) während der Kultivierung hervorgerufen, deren Ursachen unkontrollierte Wachstumsbedingungen (z.B. Licht, Temperatur bei Open-Pond- sowie geschlossenen Reaktoren) der Mikroorganismen oder die Induktion der Produktion von Wertstoffen durch die phototrophen Organismen (z.B. Astaxanthin, beta-Carotin) sein können.

Aus der WO 2008/055190 A2 ist ein geschlossener Photobioreaktor zur Kultivierung von Algen bekannt. Als Materialien werden Glas oder Kunststoffe wie Polyethylen, PET, Polycarbonat eingesetzt. In der DE 10 2005 025 118 A1 wird die Ablösung von Mikroorganismen von den Oberflächen von Bioreaktoren mittels Ultraschall beschrieben. In der US 2003/0073231 A1 und der US 2007/0048848 A1 werden die Anlagerungen mittels mechanischer Mittel beseitigt, beispielsweise Bürsten. Es handelt sich hierbei um relativ aufwändige Verfahren, welche nicht beliebig skalierbar sind. In der DE 44 16 069 A1 wird empfohlen, lichtleitende Fasern, welche zur Ausleuchtung von Bioreaktoren eingesetzt werden, mit einer glatten Oberfläche zu versehen. Die US 2008/0311649 A1 schlägt vor in Röhren-Bioreaktoren die Durchflußgeschwindigkeit des Algen enthaltenden Mediums zu erhöhen, um die Algenablagerung zu verhindern. Nachteilig ist hierbei, dass die Kultivierungsparameter bezüglich der Strömungsgeschwindigkeit nicht mehr unabhängig einstellbar sind.

In der WO 2008/132196 A1 werden vernetzbare Polyorganosiloxan-Polyoxyalkylen-Copolymere als Antifouling-Beschichtung im marinen Bereich empfohlen, insbesondere zur Beschichtung von Metall bzw. Beton, beispielsweise Schiffsrümpfe, Bojen, Bohrinseln. Weiter werden in dieser Veröffentlichung die GB 1307001 diskutiert, welche die Beschichtung von Schiffsrümpfen mit Silikonharzen zur Anwuchsverhinderung beschreibt, und die US 3702778 diskutiert, welche die Beschichtung von Schiffsrümpfen mit Silikonkautschuk beschreibt. Der WO 2008/132196 A1 ist zu entnehmen, dass in beiden Fällen eine wirksame Verhinderung des Anwuchses nur bei relativ hoher Strömungsgeschwindigkeit am Schiffsrumpf erreicht wird. Zur Verhinderung des Bewuchses von Unterwasserkonstruktionen wird in der WO 01/94487 A2 empfohlen, glasartige Interpenetrating Polymer Networks auf Basis von silanol-terminierten Silikonen und Alkoxy-funktionalisierten Siloxanen, zusammen mit zwei separierbaren Agenzien aufzubringen, von denen mindestens eines auf die Glasmatrix aufpfropft. Siliconbeschichtungen werden in dieser Schrift als in mariner Umgebung instabil beschrieben, mit dem Nachteil, dass die Beschichtung häufig erneuert werden muß. In Biofouling, 2007, 23(1), 55-62 wird empfohlen, Antifouling-Anstriche auf Siliconbasis auf Schiffsrümpfen in bestimmten Mustern aufzutragen. Anstriche, welche nur Silicone enthalten, werden als nicht inhärent anwuchshemmend beschrieben. In der WO 2008/145719 A1 werden transparente LED-Kunststoff-Formteile zur Ausleuchtung von Photoreaktoren eingesetzt. Vorzugsweise werden hierzu Formteile verwendet, bei denen LEDs in einen Silicon-Formkörper eingeschlossen sind.

In US 2007/0048859 A1 wird ein Photobioreaktor beschrieben, dessen Rohren an deren Innenseite mit einer hydrophobischen Antifouling-Beschichtung versehen sind.

Vor diesem Hintergrund bestand die Aufgabe, Bioreaktoren zur Kultivierung von Mikroorganismen dahingehend zu verbessern, dass der Bewuchs mit Mikroorganismen an den mit dem Kultivierungsmedium in Kontakt tretenden Reaktorteilen weitgehend verhindert wird, und eventuell auftretender Bewuchs ohne hohen Aufwand entfernt werden kann. Die Lösung sollte die Produktqualität nicht negativ beeinflußen, upskalierbar sein, und unabhängig von den für die Kultivierung erforderlichen Prozess-Parameter universell einsetzbar sein.

Gegenstand der Erfindung ist ein Bioreaktor zur Kultivierung von phototrophen Organismen in einem wässrigen Kultivierungsmedium, dessen mit dem Kultivierungsmedium in Kontakt kommenden Reaktorteile und/oder Reaktoreinbauten, ganz oder teilweise, mit einer Siliconschicht beschichtet sind, wobei die Oberfläche der Siliconschicht einen Kontaktwinkel zu Wasser von mindestens 100° aufweist.

Der Bioreaktor eignet sich zur Kultivierung von phototrophen Makro- oder Mikroorganismen. Als phototrophe Organismen werden dabei solche bezeichnet, welche Licht und Kohlendioxid, oder gegebenenfalls noch eine weitere Kohlenstoffquelle, zum Wachstum benötigen. Beispiele für phototrophe Makroorganismen sind Makroalgen, Pflanzen, Moose, Pflanzenzellkulturen. Beispiele für phototrophe Mikroorganismen sind phototrophe Bakterien wie Purpurbakterien und phototrophe Mikroalgen einschließlich Cyanobakterien. Bevorzugt dient der Bioreaktor der Kultivierung von phototrophen Mikroorganismen, besonders bevorzugt der Kultivierung phototropher Mikroalgen.

Bei dem Bioreaktor kann es sich um einen geschlossenen Reaktor oder einen offenen Reaktor, jeweils mit beliebiger Formgestalt handeln. Bei den offenen Reaktoren können beispielsweise Wannen oder sogenannte "open ponds" oder "raceway ponds" eingesetzt werden. Als Bioreaktoren werden geschlossene Reaktoren bevorzugt. Bei den geschlossenen Bioreaktoren kann es sich beispielsweise um Plattenbioreaktoren, Rohrbioreaktoren, (Blasen)Säulenbioreaktoren oder Schlauchbioreaktoren handeln. Plattenbioreaktoren bestehen aus senkrecht oder geneigt angeordneten quaderförmigen Platten, wobei eine Vielzahl von Platten zu einem größeren Reaktorsystem verbunden ist. Rohrbioreaktoren bestehen aus einem Rohrsystem, das senkrecht oder waagrecht oder in beliebigen Winkeln dazwischen angeordnet sein kann, wobei das Rohrsystem sehr lang sein kann, vorzugsweise bis zu mehrere hundert Kilometer. Das Kultivierungsmedium wird dabei durch das Rohrsystem gefördert, vorzugsweise mittels Pumpen oder mittels Airlift-Prinzip. Der Säulenbioreaktor besteht aus einem geschlossenen, zylindrischen Gefäß, welches mit dem Kultivierungsmedium befüllt ist. Bei Bioreaktoren dieses Typs wird ein Gemisch aus Luft und Kohlendioxid oder auch Kohlendioxid eingeleitet, wobei die aufsteigende Blasensäule für die Durchmischung des Kultivierungsmediums sorgt. Bei Schlauchreaktoren handelt es sich um ein Reaktorsystem, welches aus einem einzigen Schlauch beliebiger Länge oder aus einer Vielzahl von Schläuchen beliebiger Länge besteht.

Die Bioreaktoren werden vorzugsweise aus transparenten oder transluzenten Materialien gefertigt. Unter transparenten Materialien werden solche verstanden, welche mindestens 80 % des Lichtes im Spektralbereich von 400 nm bis 1000 nm hindurchlassen. Unter transluzenten Materialien werden solche verstanden, welche mindestens 50 % des Lichtes im Spektralbereich von 400 nm bis 1000 nm hindurchlassen. Beispielsweise Glas oder Kunststoffe wie Polymethylmethacrylat (Plexiglas), Polyester wie PET, Polycarbonat, Polyamid, Polystyrol, Polyethylen, Polypropylen, Polyvinylchlorid. Bei nicht-transparenten Photobioreaktoren können die genannten Kunststoffe, aber auch Stahl bzw. Edelstahl eingesetzt werden. Die Reaktorvolumina können beliebig gewählt werden.

Unter Reaktorteilen werden die Reaktorwandungen einschließlich Reaktorboden und Reaktorabdeckung sowie strukturgebende Elemente im Kultivierungsmedium, wie z.B. Strömungsbrecher verstanden. Den Reaktorwandungen entsprechen bei Rohr-, Platten- und Schlauchreaktoren, die Rohre, Platten und Schläuche.

Die Bioreaktoren sind mit Reaktoreinbauten ausgestattet. Beispielsweise, zur Befüllung und Nährstoffversorgung, mit Zuleitungen und zur Produktabtrennung und Entleerung mit Ableitungen. Zur Kühlung und Beheizung können die Bioreaktoren gegebenenfalls mit Heiz-/Kühlvorrichtungen wie Wärmeaustauschern ausgestattet werden. Des weiteren können die Bioreaktoren noch Rühreinrichtungen und Pumpen zur Durchmischung enthalten. Vielfach sind die Bioreaktoren noch mit Einrichtungen zur künstlichen Beleuchtung ausgestattet. Weitere Beispiele für Reaktoreinrichtungen sind Mess- und Steuergeräte für die Betriebsüberwachung.

Zur Ausrüstung von Bioreaktoren mit einer anwuchshemmenden Beschichtung geeignete Silicone sind beispielsweise kondensationsvernetzende Silicone (Siliconkautschuke), additionsvernetzende Silicone (Siliconkautschuke), Siliconhybridpolymere, Siliconharze und/oder Silicongele, sofern die Oberfläche derer Filme einen Kontaktwinkel zu Wasser von mindestens 100° aufweist. Bevorzugt werden transparente oder transluzente Silicone. Unter transparente Silicone werden dabei solche Silicone verstanden, deren Filme, als Beschichtung mit einer Schichtdicke von 10 µm, mindestens 80 % des Lichtes im Spektralbereich von 400 nm bis 1000 nm hindurchlassen. Unter transluzente Silicone werden solche verstanden, deren Filme, als Beschichtung mit einer Schichtdicke von 10 µm, mindestens 50 % des Lichtes im Spektralbereich von 400 nm bis 1000 nm hindurchlassen.

Kondensationsvernetzende Siliconkautschuk-Systeme enthalten
a) kondensationsfähige Endgruppen aufweisende Organopolysiloxane,
b) gegebenenfalls je Molekül mindestens drei an Silicium gebundene hydrolysierbare Gruppen aufweisende Organosiliciumverbindungen, sowie
c) Kondensationskatalysatoren.

Geeignete vernetzte Siliconkautschuke, welche durch Kondensationsreaktion vernetzen, sind bei Raumtemperatur vernetzende 1-Komponenten-Systeme, sogenannte RTV-1-Silikonkautschuke. Bei den RTV-1-Siliconkautschuken handelt es sich um Organopolysiloxane mit kondensationsfähigen Endgruppen, welche in Gegenwart von Katalysatoren unter Kondensation bei Raumtemperatur vernetzen. Am gebräuchlichsten sind Dialkylpolysiloxane der Struktur R₃SiO[-SiR₂O]ₙ-SiR₃ mit einer Kettenlänge von n > 2. Die Alkylreste R können gleich oder verschieden sein und haben im allgemeinen 1 bis 4 C-Atome und können gegebenenfalls substituiert sein. Die Alkylreste R können auch teilweise durch andere Reste ersetzt sein, vorzugsweise durch Arylreste, welche gegebenenfalls substituiert sind, und wobei die Alkyl-(Aryl)-Gruppen R teilweise durch zur Kondensations-Vernetzung fähige Gruppen ausgetauscht sind, beispielsweise Alkohol- (Alkoxysystem), Acetat- (Essigsäuresystem), Amin- (Aminsystem) oder Oximreste (Oximsystem). Die Vernetzung wird mittels geeigneter Katalysatoren, beispielsweise Zinn- oder Titankatalysatoren katalysiert.

Geeignete vernetzte Siliconkautschuke, welche durch Kondensationsreaktion vernetzen, sind auch bei Raumtemperatur vernetzende 2-Komponenten-Systeme, sogenannte RTV-2-Silikonkautschuke. RTV-2-Siliconkautschuke erhält man mittels Kondensations-vernetzung von mehrfach mit Hydroxygruppen substituierten Organopolysiloxanen in Gegenwart von Kieselsäureestern. Als Vernetzer können auch Alkylsilane mit Alkoxy- (Alkoxysystem), Oxim-(Oximsystem), Amin- (Aminsystem) oder Acetatgruppen (Essigsäuresystem) eingesetzt werden, welche in Anwesenheit von geeigneten Kondensations-Katalysatoren, beispielsweise Zinn- oder Titankatalysatoren mit den Hydroxygruppen-terminierten Polydialkylsiloxanen vernetzen.

Beispiele für die in RTV-1 und RTV-2 Siliconkautschuk enthaltenen Polydialkylsiloxane sind solche der Formel (OH)R₂SiO[-SiR₂O]ₙ-SiR₂(OH) mit einer Kettenlänge von n > 2, wobei die Alkylreste R gleich oder verschieden sein können, und die Reste R die oben angegebene Bedeutung haben. Vorzugsweise enthalten die Polydialkylsiloxane terminale OH-Gruppen, welche mit den Kieselsäureestern bzw. dem System Alkylsilan/Zinn(Titan)-katalysator bei Raumtemperatur vernetzen.

Beispiele für die in RTV-1 und RTV-2 Siliconkautschuken enthaltenen, hydrolysierbare Gruppen aufweisende Alkylsilane sind solche der Formel RₐSi(OX)₄₋ₐ, mit a = 1 bis 3 (bevorzugt 1), und X in der Bedeutung von R' (Alkoxysystem), C(O)R' (Essigsäuresystem), N=CR'₂ (Oximsystem) oder NR'₂ (Aminsystem), wobei R' einen einwertigen Kohlenwasserstoffrest mit 1 bis 6 Kohlenstoffatomen bedeutet.

Additionsvernetzende Siliconkautschuk-Systeme enthalten
a) Organosiliciumverbindungen, die Reste mit aliphatischen Kohlenstoff-Kohlenstoff-Mehrfachbindungen aufweisen,
b) gegebenenfalls Organosiliciumverbindungen mit Si-gebundenen Wasserstoffatomen oder anstelle von a) und b)
c) Organosiliciumverbindungen, die Reste mit aliphatischen Kohlenstoff-Kohlenstoff-Mehrfachbindungen und Si-gebundene Wasserstoffatome aufweisen,
d) die Anlagerung von Si-gebundenen Wasserstoff an aliphatische Mehrfachbindung fördernde Katalysatoren und
e) gegebenenfalls die Anlagerung von Si-gebundenem Wasserstoff an aliphatische Mehrfachbindung bei Raumtemperatur verzögernde Mittel.

Geeignete additionsvernetzte Siliconkautschuke sind bei Raumtemperatur vernetzende 1-Komponenten-Systeme, sogenannte additionsvernetzende RTV-1-Siliconkautschuke, bei Raumtemperatur vernetzende 2-Komponenten-Systeme, sogenannte additionsvernetzende RTV-2-Silikonkautschuke oder auch bei Raumtemperatur vernetzende Mehrkomponenten-Systeme. Die Vernetzungsreaktion kann dabei kationisch, mittels entsprechender Katalysatoren, oder radikalisch, mittels Peroxiden, oder durch Strahlung, insbesondere UV-Strahlung, oder thermisch initiiert werden.

Additionsvernetzende RTV-2-Siliconkautschuke erhält man durch mit Pt-Katalysatoren katalysierte Vernetzung von mehrfach ethylenisch ungesättigten Gruppen, vorzugsweise Vinylgruppen, substituierten Organopolysiloxanen mit mehrfach mit Si-H-Gruppen substituierten Organopolysiloxanen in Gegenwart von Platinkatalysatoren.

Vorzugsweise besteht eine der Komponenten aus Dialkylpolysiloxanen der Struktur R₃SiO[-SiR₂O]ₙ-SiR₃ mit n ≥ 0, wobei die Reste R die oben angegebene Bedeutung haben. Im allgemeinen ist R ein Alkylrest mit 1 bis 4 C-Atomen, wobei die Alkylreste ganz oder teilweise durch Arylreste wie den Phenylrest ersetzt werden können, und an einem oder an beiden Enden einer der endständigen Reste R durch eine polymerisierbare Gruppe wie die Vinylgruppe ersetzt ist. Ebenso können teilweise Reste R in der Siloxankette, auch in Kombination mit den Resten R der Endgruppen, durch polymerisierbare Gruppen ersetzt werden. Bevorzugt werden vinylendblockierte Polydimethylsiloxane der Struktur (CH₂=CH₂)R₂SiO[-SiR₂O]ₙ-SiR₂(CH₂=CH₂) eingesetzt.

Die zweite Komponente enthält einen Si-H-funktionellen Vernetzer. Die üblicherweise verwendeten Polyalkylhydrogensiloxane sind Copolymere aus Dialkylpolysiloxanen und Polyalkylhydrogensiloxanen mit der allgemeinen Formel R''₃SiO[-SiR₂O]ₙ-[SiHRO]ₘ-SiR''₃ mit m ≥ 0, n ≥ 0 und der Maßgabe, dass mindestens zwei SiH-Gruppen enthalten sein müssen, wobei R'' die Bedeutung von H oder R haben kann. Es gibt demnach Vernetzer mit seitenständigen und endständigen SiH-Gruppen, während Siloxane mit R'' = H, die nur endständige SiH-Gruppen besitzen, auch noch zur Kettenverlängerung verwendet werden. Als Vernetzungskatalysator sind geringe Mengen einer platinorganischen Verbindung enthalten.

In jüngster Zeit sind zudem noch spezielle Siliconkautschuke im Handel erhältlich, welche über die beschriebene Additionsreaktion vernetzt werden, indem spezielle Platin-Komplexe bzw. Platin-/Inhibitor-Systeme thermisch und/oder photochemisch aktiviert werden und somit die Vernetzungsreaktion katalysieren.

Geeignete Materialien sind auch Siliconhybridpolymere. Siliconhybridpolymere sind Copolymerisate oder Pfropfcopolymerisate von Organopolymerblöcken, beispielsweise Polyurethan, Polyharnstoff oder Polyvinylestern, und Siliconblöcken, im allgemeinen auf Basis von Polydialkylsiloxanen der obengenannten Spezifikation. Beispielsweise werden thermoplastische Siliconhybridpolymere in EP 1412416 B1 und EP 1489129 B1 beschrieben, deren diesbezügliche Offenbarung auch Gegenstand dieser Anmeldung sein soll. Derartige Siliconhybridpolymere werden als Thermoplastische Siliconelastomere (TPSE) bezeichnet und sind im Handel erhältlich. Geeignete Materialien sind auch (Kondensations- oder Strahlungs-) vernetzbare Siliconhybridmaterialien, wie in WO 2006/058656 beschrieben, deren diesbezügliche Angaben Teil dieser Anmeldung sind (icorporated by reference).

Siliconharze sind ebenfalls geeignete Materialien für die Herstellung der transparenten oder transluzenten Beschichtung. Im allgemeinen enthalten die Siliconharze Einheiten der allgemeinen Formel R_{b}(RO)_{c}SiO_{(4-b-c)/2}, worin b gleich 0, 1, 2 oder 3 ist, c gleich 0, 1, 2 oder 3 ist, mit der Maßgabe, dass b+c ≤ 3 ist, und R in der oben dafür angegebenen Bedeutung, welche ein hoch vernetztes Organosilicon-Netzwerk aufbauen. Siliconharze können lösemittelfrei, lösemittelhaltig oder als wässrige Systeme zum Einsatz kommen. Darüberhinaus können auch funktionalisierte, z.B. mit Epoxy- oder Amingruppen funktionalisierte Siliconharze eingesetzt werden.

Silicongele sind ebenfalls geeignete Materialien für die Herstellung der transparenten oder transluzenten Beschichtung. Silicongele werden aus zwei gießbaren Komponenten hergestellt, welche bei Raumtemperatur in Gegenwart eines Katalysators vernetzen. Eine der Komponenten besteht im allgemeinen aus Dialkylpolysiloxanen der Struktur R₃SiO[-SiR₂O]ₙ-SiR₃ mit n ≥ 0 und R in der oben dafür angegebenen Bedeutung, im allgemeinen mit 1 bis 4 C-Atomen im Alkylrest, wobei die Alkylreste ganz oder teilweise durch Arylreste wie den Phenylrest ersetzt werden können, und an einem oder an beiden Enden einer der endständigen Reste R durch eine polymerisierbare Gruppe wie die Vinylgruppe ersetzt ist. Ebenso können teilweise Reste R in der Siloxankette, auch in Kombination mit den Resten R der Endgruppen, durch polymerisierbare Gruppen ersetzt werden. Bevorzugt werden vinylendblockierte Polydimethylsiloxane der Struktur (CH₂=CH₂)R₂SiO[-SiR₂O]ₙ-SiR₂(CH₂=CH₂) eingesetzt.

Die zweite Komponente enthält einen Si-H-funktionellen Vernetzer. Die üblicherweise verwendeten Polyalkylhydrogensiloxane sind Copolymere aus Dialkylpolysiloxanen und Polyalkylhydrogensiloxanen mit der allgemeinen Formel R''₃SiO[-SiR₂O]ₙ-[SiHRO]ₘ-SiR''₃ mit m ≥ 0, n ≥ 0 und der Maßgabe, dass mindestens zwei SiH-Gruppen enthalten sein müssen, wobei R'' die Bedeutung von H oder R haben kann. Es gibt demnach Vernetzer mit seitenständigen und endständigen SiH-Gruppen, während Siloxane mit R'' = H, die nur endständige SiH-Gruppen besitzen, auch noch zur Kettenverlängerung verwendet werden. Als Vernetzungskatalysator sind geringe Mengen einer platinorganischen Verbindung enthalten. Durch das Mischen der Komponenten wird die Vernetzungsreaktion ausgelöst und das Gel gebildet. Diese Vernetzungsreaktion kann durch das Einwirken von Wärme und/oder durch elektromagnetische Strahlung, vorzugsweise UV-Strahlung, beschleunigt werden.

Eine detaillierte Übersicht über Silicone, ihre Chemie, Formulierung und Anwendungseigenschaften findet sich beispielsweise in Winnacker/Küchler, "Chemische Technik: Prozesse und Produkte, Band 5: Organische Zwischenverbindungen, Polymere", Seite 1095-1213, Wiley-VCH Weinheim (2005).

Wesentlich für die Hemmung bzw. Verhinderung des Bewuchses mit Mikroorganismen ist die Morphologie der Oberfläche der Siliconbeschichtung. Die Morphologie der Oberfläche wird über den Kontaktwinkel dieser Oberfläche zu Wasser bestimmt. Der erfindungsgemäße Kontaktwinkel wird durch die erfindungsgemäße Auswahl der Siliconmaterialien eingestellt. Auf weitere Maßnahmen zur Erhöhung des Kontaktwinkels, beispielweise Aufrauhung der Oberfläche (z.B. zur Nachahmung des sog. Lotuseffekts), wird vorzugsweise verzichtet. Eine solche Aufrauhung kann nämlich die Kultivierung der phototrophen Mikroorganismen stören. Bevorzugt sind Oberflächen mit Kontaktwinkeln zwischen 100° und 120°, besonders bevorzugt Oberflächen mit Kontaktwinkeln zwischen 100° und 115°, und ganz besonders bevorzugt Oberflächen mit Kontaktwinkeln zwischen 100° und 113°. Die Bestimmung des Kontaktwinkels der Oberfläche der Siliconbeschichtung zu Wasser kann mittels dem Fachmann bekannter Methoden, beispielsweise gemäß DIN 55660-2 erfolgen, unter Einsatz von im Handel erhältlicher Messgeräte zur Bestimmung des Kontaktwinkels, beispielsweise den bei der Fa. Krüss erhältlichen Kontaktwinkel-Messsystemen.

Die mit dem Kultivierungsmedium in Kontakt kommenden Reaktorteile, insbesondere die Innenseiten der Reaktorwandungen, werden vollständig oder teilweise, vorzugsweise vollständig, mit den genannten Siliconen beschichtet. In einer bevorzugten Ausführungsform werden auch die Reaktoreinbauten ganz oder teilweise mit Silicon beschichtet. Die Silicone werden in flüssiger Form, entweder in Reinsubstanz, als Lösung oder in wässeriger Emulsion aufgetragen. Vorzugsweise beträgt die Viskosität der zur Beschichtung aufzutragenden Flüssigkeit von 10 mPas bis 300.000 mPas.

Den Siliconen werden keine Zusatzstoffe beigegeben, welche aus der Beschichtung freigesetzt werden können, wie dies in marinen Antifouling-Systemen üblich ist. Die Auftragung kann mittels der üblichen Techniken erfolgen, vorzugsweise Streichen, Sprühen, Tauchen, Rakeln, Gießen. Besonders bevorzugt ist hierbei das Tauchen und Sprühen. Es können aber zur Beschichtung von Rohren auch noch weitere Verfahren wie z.B. Schwammauftrag, Schleudern, Extrusion bzw. Querkopfextrusion angewandt werden, sowie für ebene Flächen zusätzlich noch Auftragung mittels Walzenbeschichtung, Rollenbeschichtung oder Pflatschen.

Die Auftragung erfolgt vorzugsweise direkt auf die Reaktorteile bzw. Reaktoreinbauten, ohne Auftragung einer Primerschicht. Im Allgemeinen beträgt die Dicke der Beschichtung 10 nm bis 1000 µm, vorzugsweise 1 µm bis 100 µm. Gegebenenfalls können die zu beschichtenden Reaktorteile, zur Verbesserung der Haftung der Silicone, vorbehandelt werden, beispielsweise mittels Corona-Behandlung. Gegebenenfalls können die Silicone übliche Additive zur Haftvermittlung oder übliche Füllstoffe zur Verbesserung der Mechanik enthalten. Diese Additive werden vorzugsweise maximal in solchen Mengen eingesetzt, dass die Siliconbeschichtung transparent bzw. transluzent bleibt.

Gegebenenfalls an den beschichteten Flächen anhaftende Organismen können zwischen den Kultivierungszyklen durch Absprühen beispielsweise mit Wasser, Ethanol oder H₂O₂ ohne weitere mechanische Bearbeitung entfernt werden.

Die erfindungsgemäß mit Silicon beschichteten Photobioreaktoren minimieren die Ablagerung der sich bildenden phototrophen Organismen, so dass die Strömungsbedingungen des Kultivierungsmediums konstant bleiben, und die für das Wachstum ideale Lichteintragsmenge auf maximales Wachstum der zu kultivierenden Organismen eingestellt bleibt. Außerdem wird eine Minimierung des Reinigungsaufwands zwischen einzelnen Kultivierungszyklen sowie bei einem Wechsel der zu kultivierenden phototrophen Organismen erhalten. Dies führt zu wesentlichen ökonomischen Vorteilen aufgrund kürzerer Stillstandzeiten und geringerem Reinigungsaufwand.

Gegenüber herkömmlichen siliconhaltigen Foul-Release-Beschichtungen, wie sie beispielsweise zur Beschichtung von Schiffsrümpfen eingesetzt werden, zeichnen sich die erfindungsgemäß eingesetzten Silicone dadurch aus, dass auf Release-Substanzen, welche aus der Beschichtung freigesetzt werden (z.B. Silikonöl), verzichtet wird. Darüberhinaus kann auf die Auftragung von Zwischenschichten (Grundierung) für bessere Haftung und Mechanik der in Foul-Release-Beschichtungen eingesetzten Rezepturen verzichtet werden. Die Realisierung von Kontaktwinkeln zu Wasser mit Werten von mindestens 100°, mittels Einsatz von entsprechenden Siliconmaterialien reduziert zum einen die Anlagerung von Wasser auf der Siliconoberfläche, zum anderen werden auch in Wasser gelöste Stoffe, die z.B. durch Stresssituationen während der Algenkultivierung entstehen, von der Oberfläche ferngehalten.

## Patentansprüche

1. Bioreaktor, **gefertigt aus einem Material aus der Gruppe bestehend aus Glas, Polymethylmethacrylat, Polyester, Polycarbonat, Polyamid, Polystyrol, Polyethylen, Polyvinylchlorid, Stahl und Edelstahl,** zur Kultivierung von phototrophen Organismen in einem wässrigen Kultivierungsmedium, dessen mit dem Kultivierungsmedium in Kontakt kommenden Reaktorteile, ganz oder teilweise, mit einer Siliconschicht beschichtet sind, wobei die Oberfläche der Siliconschicht einen Kontaktwinkel zu Wasser von mindestens 100° aufweist.

2. Bioreaktor nach Anspruch 1, **dadurch gekennzeichnet, dass** der Bioreaktor ein geschlossener Reaktor ist.

3. Bioreaktor nach Anspruch 1, **dadurch gekennzeichnet, dass** der Bioreaktor ein Plattenbioreaktor, Rohrbioreaktor, (Blasen)Säulenbioreaktor oder Schlauchbioreaktor ist.

4. Bioreaktor nach Anspruch 1 bis 3, **dadurch gekennzeichnet, dass** der Bioreaktor aus transparenten oder transluzenten Materialien gefertigt ist.

5. Bioreaktor nach Anspruch 1 bis 4, **dadurch gekennzeichnet, dass** die Reaktorteile mit einer transparenten oder transluzenten Siliconschicht beschichtet sind.

6. Bioreaktor nach Anspruch 1 bis 5, **dadurch gekennzeichnet, dass** die Siliconschicht ein oder mehrere Silicone enthält aus der Gruppe enthaltend kondensationsvernetzende Siliconelastomere, additionsvernetzende Siliconelastomere, Siliconhybridpolymere, Siliconharze sowie Silicongele.

7. Verfahren zur Ausrüstung von Bioreaktoren mit einer anwuchshemmenden Beschichtung, wobei der Bioreaktor an den mit dem Kultivierungsmedium in Kontakt kommenden Reaktorteilen, ganz oder teilweise, mit einer Siliconschicht beschichtet wird und die Oberfläche der Siliconschicht einen Kontaktwinkel zu Wasser von mindestens 100° aufweist.

8. Verwendung von Siliconen, deren Oberfläche bei Auftragung als Film einen Kontaktwinkel zu Wasser von mindestens 100° aufweist, zur Ausrüstung von Bioreaktoren mit einer anwuchshemmenden Beschichtung.

## Claims

1. Bioreactor, manufactured from a material from the group consisting of glas, polymethylmethacrylate, polyester, polycarbonate, polyamide, polystyrene, polytheylene, polyvinyl chloride, steel and stainless steel, for cultivating phototrophic organisms in an aqueous culture medium, in which the reactor parts that come into contact with the culture medium are coated completely or partially with a silicone layer, wherein the surface of the silicone layer has a contact angle to water of at leat 100°.

2. Bioreactor according to Claim 1, **characterized in that** the bioreactor is a closed reactor.

3. Bioreactor according to Claim 1, **characterized in that** the bioreactor is a plate-type bioreactor, tubular bioreactor, (bubble) column bioreactor or hose-type bioreactor.

4. Bioreactor according to Claims 1 to 3, **characterized in that** the bioreactor is made of transparent or translucent materials.

5. Bioreactor according to Claims 1 to 4, **characterized in that** the reactor parts are coated with a transparent or translucent silicone layer.

6. Bioreactor according to Claims 1 to 5, **characterized in that** the silicone layer contains one or more silicones from the group containing condensation-crosslinking silicone elastomers, addition-crosslinking silicone elastomers, silicone hybrid polymers, silicone resins and silicone gels.

7. Method for providing bioreactors with an antifouling coating, wherein the bioreactor is coated, on the reactor parts coming into contact with the culture medium, completely or partially with a silicone layer, and the surface of the silicone layer has a contact angle to water of at least 100°.

8. Use of silicones having a surface which, when applied as a film, has a contact angle to water of at least 100° for providing bioreactors with an antifouling coating.

## Revendications

1. Bioréacteur, fabriqué en un matériau du groupe composé de verre, polyméthylméthacrylate, polyester, polycarbonate, polyamide, polystyrène, polyéthylène, chlorure de polyvinyle, acier et acier allié, pour la culture d'organismes phototrophes dans un milieu de culture aqueux, dont les parties de réacteur venant en contact avec le milieu de culture sont revêtues entièrement ou partiellement d'une couche de silicone, dans lequel la surface de la couche de silicone présente un angle de contact avec l'eau d'au moins 100°.

2. Bioréacteur selon la revendication 1, **caractérisé en ce que** le bioréacteur est un réacteur fermé.

3. Bioréacteur selon la revendication 1, **caractérisé en ce que** le bioréacteur est un bioréacteur à plaques, un bioréacteur à tubes, un bioréacteur à colonnes (à bulles) ou un bioréacteur à tuyau flexible.

4. Bioréacteur selon l'une quelconque des revendications 1 à 3, **caractérisé en ce que** le bioréacteur est fabriqué en matériaux transparents ou translucides.

5. Bioréacteur selon l'une quelconque des revendications 1 à 4, **caractérisé en ce que** les parties de réacteur sont revêtues d'une couche de silicone transparente ou translucide.

6. Bioréacteur selon l'une quelconque des revendications 1 à 5, **caractérisé en ce que** la couche de silicone contient un ou plusieurs silicones du groupe comprenant des élastomères silicones à réticulation par condensation, des élastomères silicones à réticulation par addition, des polymères hybrides de silicone, des résines de silicone ainsi que des gels de silicone.

7. Procédé pour équiper des bioréacteurs avec un revêtement antisalissure, dans lequel le bioréacteur est revêtu, entièrement ou partiellement, d'une couche de silicone sur les parties de réacteur venant en contact avec le milieu de culture, et la surface de la couche de silicone présente un angle de contact avec l'eau d'au moins 100°.

8. Utilisation de silicones, dont la surface présente, lors d'un dépôt sous forme de film, un angle de contact avec l'eau d'au moins 100°, pour l'équipement de bioréacteurs avec un revêtement antisalissure.
